# EUROPÄISCHE PATENTSCHRIFT

(11) **EP 1 818 323 B1**
(45) Veröffentlichungstag und Bekanntmachung des Hinweises auf die Patenterteilung: **30.03.2011**
(21) Anmeldenummer: 07002270.2
(22) Anmeldetag: 02.02.2007
(51) Int. Cl.: C07C 41/16, C07C 43/11, C08G 65/00, C08G 65/34, C08G 65/32, C08G 65/329, C08G 65/332, C08G 65/337

(54) **Verfahren zur Herstellung von unsymmetrischen alkoxylierten Mischethern**
Process for the preparation of unsymmetric alkoxylated mixed ethers
Procédé de préparation d'éthers mixtes alkoxylés non symétriques

(30) Priorität: 08.02.2006 DE 102006005727
(43) Veröffentlichungstag der Anmeldung: 15.08.2007
(73) Patentinhaber: Cognis IP Management GmbH, 40589 Düsseldorf (DE)
(72) Erfinder: Mahnke, Eike Ulf, 42553 Velbert (DE); Rößler, Harald, 40593 Düsseldorf (DE); Huesken, Hendrik, 45721 Haltern am See (DE)

(56) Entgegenhaltungen:
- DE-A1- 4 333 101
- US-A- 4 061 682
- GROBELNY Z ET AL: "Decomposition of vinyl ethers by alkalide K<->, K<+>(15-crown-5)2 via organopotassium intermediates" JOURNAL OF ORGANOMETALLIC CHEMISTRY, ELSEVIER-SEQUOIA S.A. LAUSANNE, CH, Bd. 689, Nr. 9, 1. Mai 2004 (2004-05-01), Seiten 1580-1585, XP004502201 ISSN: 0022-328X
- J. OF MOLECULAR STRUCTURE, Nr. 444, 1998, Seiten 69-76, XP008081801
- FREEDMAN, H. H.; DUBOIS, R. A.: "An improved Williamson ether synthesis using phase transfer catalysis" TETRAHEDRON LETTERS, Nr. 38, 1975, Seiten 3251-3254, XP002444500

## Beschreibung

### Gebiet der Erfindung

Die Erfindung befindet sich auf dem Gebiet der nichtionischen Tenside und betrifft ein verbessertes Verfahren zur Herstellung spezieller Mischether mit Hilfe von Phasen Transfer Katalysatoren.

### Stand der Technik

Nichtionische Tenside vom Typ der Alkylpolyglycolether werden aus den entsprechenden Alkoholen durch Umsetzung mit Alkylenoxiden, speziell Ethylen- oder Propylenoxid im Molverhältnis 1:2 bis 1:12 hergestellt. Die Umsetzung erfolgt dabei unter den bekannten Alkoxylierungsbedingungen, vorzugsweise in Gegenwart von geeigneten alkalischen Katalysatoren.

Zur Herstellung der so genannten alkoxylierten Mischether, einer weiteren Klasse von Niotensiden, die sich durch besondere Schaumarmut auszeichnen, werden die in den Alkylpolyglycolethern vorliegenden Hydroxylgruppen verethert, was bevorzugt unter den Bedingungen der Williamson'schen Ethersynthese bei üblicherweise 120°C mit geradkettigen oder verzweigten C₄-C₈ Alkyl- oder Arylhalogeniden durchgeführt wird, wie beispielsweise mit n-Butyliodid, n-Butylchlorid, sec.-Butylbromid, tert.-Butylchlorid, Amylchlorid, tert.-Amylbromid, n-Hexylchlorid, n-Heptylbromid, n-Octylchlorid oder Benzylchlorid. Dabei werden Halogenid und Alkali üblicherweise bezogen auf die zu verethernden Hydroxylgruppen im hohem stöchiometrischen Überschuss eingesetzt [DE 33 15 951 A1].

Gegenstand der DE-OS 2800710 (Kuraray)aus dem Jahre 1978 ist ein Verfahren zur Herstellung von Polyglykoletherderivaten, bei dem man "reine" Polyglykolether bzw. Polyglykolether, die einseitig mit Stickstoffsubstituenten verschlossen sind, mit organischen Halogenverbindungen in Gegenwart von wässrigen Alkalibasen umsetzt. Die Lehre löst dabei die Aufgabe, bestehende Verfahren zur Herstellung von endgruppenverschlossenen Polyglykolethern zu verbessern. Zu diesem Zweck wurde das nach dem Stand der Technik bisher übliche Verfahren, bei dem zunächst die Polyglykoletherverbindung in das Alkoholat überführt und dieses dann mit der organischen Halogenverbindung verethert wird. Die Verwendung von wässrigen Basen anstelle von metallischem Natrium, Hydridverbindungen oder Alkalialkoholaten führt zu einer Verbilligung des Verfahrens und einer Steigerung der Ausbeute. Seit den 80er Jahren stellt das Kuraray-Verfahren daher die Methode der Wahl dar, wenn es darum geht so genannte Mischetherverbindungen herzustellen

Symmetrische Ether werden mit hohen Ausbeuten hergestellt, indem man ein Alkylhalogenid, -sulfat oder -sulfonat mit Basen in Gegenwart einer katalytischen Menge eines Ammonium- oder Phosphoniumsalzes und eines Carboxylatsalzes umsetzt. Beispielsweise wird gemäß der US 4,061,682 Dibenzylether mit Ausbeuten von mehr als 95 % der Theorie durch die Reaktion von Benzylchlorid mit einer 50 Gew.-%igen Natriumhydroxid-Lösung in Gegenwart einer katalytischen Menge tetra-n-Butylammoniumbisulfat und Natriumacetat erhalten. Unsymmetrische Ether können auf diese Weise jedoch nicht hergestellt werden.

In Tetrahedron Letters, 3251 (1975**)** wird beschrieben, dass man niedermolekulare einwertige Alkohole mittels einer Phasen Transfer Katalyse in die gewünschten unsymmetrischen Ether überführen kann. Hinweise oder gar Beispiele für die Umsetzung mehrfach alkoxylierter Alkohole zu den entsprechenden Ethern finden sich in diesem Aufsatz jedoch nicht.

Die Verfahren des Stands der Technik weisen eine Reihe von Nachteilen auf: So ist es beispielsweise erforderlich, die Veretherung bei vergleichsweise hohen Temperaturen durchzuführen, da ansonsten keine befriedigenden Ausbeuten und Umsätze zu erzielen sind. Abgesehen von den Kosten eines hohen Energieeintrages begünstigt die hohe Reaktionstemperatur zudem auch unerwünschte Nebenreaktionen, wie insbesondere Eliminerungen am Alkylierungsmittel. Weiterhin von Nachteil ist, dass verzweigte Einsatzmaterialien, wie beispielsweise ethoxylierte Guerbetalkohole praktisch gar nicht umgesetzt werden können. Schließlich ist die Herstellung von wasserfreien Alkoholaten, die als alkalische Katalysatoren üblicherweise eingesetzt werden, technisch aufwendig und trägt zu den Herstellungskosten nicht unerheblich bei.

Die komplexe Aufgabe der vorliegenden Erfindung hat somit darin bestanden, ein neues Herstellverfahren für Mischether auf Basis von ethoxylierten Guerbetalkoholen zur Verfügung zu stellen, welches frei von den eingangs geschilderten Nachteilen des Stands der Technik ist und insbesondere bei niedrigen Reaktionstemperaturen hohe Ausbeuten und Umsätze möglich macht, so dass Nebenreaktionen vermieden werden können. Schließlich sollte es ohne die Verwendung von wasserfreien Alkoholatkatalysatoren auskommen.

### Beschreibung der Erfindung

Gegenstand der Erfindung ist ein Verfahren zur Herstellung von unsymmetrischen alkoxylierten Mischethern der Formel (1)

R¹O(CH₂CH₂O)ₙ₁(CH₂CHR²O)ₘ(CH₂CH₂O)ₙ₂R³ (I)

in der R¹ für einen verzweigten Alkylrest mit 12 bis 16 Kohlenstoffatomen, R² für Methyl, R³ für einen Butylrest, n1 und n2 unabhängig voneinander für 0 oder Zahlen von 5 bis 15 sowie m für 0 oder Zahlen von 1 bis 20 mit der Maßgabe steht, dass die Summe (n1+n2+m) von 0 verschieden ist, durch Veretherung von Alkylpolyglykolethern der Formel (II)

R¹O(CH₂CH₂O)ₙ₁(CH₂CHR²O)ₘ(CH₂CH₂O)ₙ₂H (II)

in der R¹, R², n1, n2 und m die oben angegebene Bedeutung haben, mit 1-Chlorbutan dadurch gekennzeichnet, dass man die Umsetzung in Gegenwart von Phasen Transfer Katalysatoren (PTK) und Basen durchführt.

Überraschenderweise wurde gefunden, dass bei der Herstellung von unsymmetrischen Mischethern auf Basis von alkoxylierten Guerbetalkoholen unter den Bedingungen einer Williamson'schen Ethersynthese mit einem Phasen Transfer Katalysator die Reaktionstemperatur deutlich niedriger gewählt werden kann als dies bei der nach dem Stand der Technik sonst üblichen alkalischen Katalyse möglich ist. Diese erniedrigte Reaktionstemperatur von insbesondere 40 bis 80 °C führt zu einer Verlangsamung bzw. Unterdrückung der Nebenreaktionen, insbesondere der Eliminierungsreaktion am Alkylierungsmittel. Ohne den Zusatz der PTK ist bei einer solch niedrigen Temperatur die gewünschte Reaktion nicht zu beobachten. Bei der Anwendung des erfindungsgemäßen Verfahrens werden trotz sehr kurzer Reaktionszeiten sehr hohe Umsätze und Ausbeuten erzielt. Die Reaktion findet im Zweiphasensystem aus wässrigem Alkalihydroxid und Alkylierungsmittel mit alkoxyliertem Alkohol statt. Auf diesem Wege lassen sich gerade auch verzweigte ethoxylierte oder propoxylierte Alkohole, die so genannten ethoxylierten oder propoxylierten Guerbetalkohole umsetzen. Ein weiterer Vorteil besteht ferner darin, dass auf die technisch und energetisch aufwendige Herstellung des wasserfreien Alkoholat-Katalysators verzichtet werden kann.

### Alkylpolyglykolether

Als Ausgangsstoffe kommen die Anlagerungsprodukte von durchschnittlich 5 bis 15 Mol Ethylenoxid und/oder 1 bis 20, vorzugsweise 2 bis 5 Mol Propylenoxid an Guerbetalkohole, speziell Hexyldecanol und Octyldodecanol in Betracht. Sofern die alkoxylierten Alkohole sowohl Ethylen- als auch Propylenglykoleinheiten aufweisen, können diese blockweise oder statistisch ("random") über die Polyetherkette verteilt sein.

### Alkylierungsmittel

Als Alkylierungsmittel wird 1-Chlorbutan ("Butylchlorid") eingesetzt. Das Alkylierungsmittel wird wie bei Willaimson-Synthesen üblich im Überschuss eingesetzt, wobei vorzugsweise ein molares Verhältnis Alkylpolyglykolether:Alkylierungsmittel von 1:1 bis 1:1,5 und insbesondere 1:1,1 bis 1:1,2 eingestellt wird.

### Phasen Transfer Katalysatoren

Als Phasen Transfer Katalysatoren können praktisch alle PTK verwendet werden, die in der Literatur, insbesondere in J.Am.Chem.Soc., 93, 195, (1971**)** sowie GB 1,227,144 A1 beschrieben werden. Die Lehre dieser beiden Schriften wird daher durch Referenz mit umfasst. Typische Beispiele für geeignete PTK sind die Gruppen der Tetraalkylammoniumsalze, Benzyltrialkylammoniumsalze, Tetraalkylphosphoniumsalze, Benzyltrialkylphosphoniumsalze und deren Gemische. Im Sinne des erfindungsgemäßen Verfahrens sind die Ammoniumsalze gegenüber den Phosphoniumsalzen bevorzugt und insbesondere finden die Tetra-n-butylammonium-, Tri-n-butylmethylammonium- und Benzyltriethylammoniumsalze mit den Anionen Chlorid, Bromid und Hydrogensulfat Anwendung. Der Phasen Transfer Katalysator wird in katalytischen Mengen zugesetzt. Die Menge hängt dabei von der Aktivität und Stabilität des Katalysators bei den gewählten Reaktionsbedingungen ab. Die Menge kann dabei zwischen 0,1 und 25 Mol-% - bezogen auf die Alkylpolyglykolether - variieren und liegt besonders bevorzugt im Bereich von 1 bis 6 Mol-%.

Ebenso können ionische Flüssigkeiten ("ionic liquids") für die Phasen Transfer Katalyse eingesetzt werden. Als ionische Flüssigkeiten bezeichnet man bei niedrigen Temperaturen (kleiner 100 °C) schmelzende Salze, die eine neuartige Klasse von Chemikalien mit nichtmolekularem, ionischem Charakter darstellen. Im Gegensatz zu klassischen Salzschmelzen, die hochschmelzende, hochviskose uns sehr korrosive Medien darstellen, sind ionische Flüssigkeiten bereits bei niedrigen Temperaturen flüssig und relativ niedrig viskos [vgl. K.R. Seddon, J.Chem.Technol.Biotechnol. 68, 351-356 (1997**)**]. Ionische Flüssigkeiten bestehen in den meisten Fällen aus Anionen wie Halogeniden, Carboxylaten, Phosohaten, Sulfaten, Sulfonaten, Tetrafluoroboraten, Hexafluorophosphaten etc. kombiniert mit substituierten Ammonium-, Phosphonium-, Pyridinium- oder Imidazolium-Kationen. Einige klassische Phasen Transfer Katalysatoren (z.B. Aliquat• 336 von Cognis Deutschland GmbH & Co. KG) können aufgrund der chemischen Struktur und physikalischen Eigenschaften auch als ionische Flüssigkeit bezeichnet werden.

Neben den Phasen Transfer Katalysatoren können auch noch Co-Katalysatoren mit verwendet werden. Als Co-Katalysatoren werden Carbonate, Carboxylate und Thiocarboxylate eingesetzt, wobei die Anionen alkyl-, aryl-, aralkyl-, cycloalkyl- oder auch substituierte Reste aufweisen können. Die Kettenlänge kann dabei zwischen 1 und 18 Kohlenstoffatomen variieren. Als Kationen können nahezu alle erdenklichen Kationen verwendet werden, insbesondere Alkali- oder Erdalkalimetallionen. Die Einsatzmenge der Co-Katalysatoren kann bezogen auf die PTK 1 bis 10 und vorzugsweise 2 bis 5 Gew.-% betragen.

### Durchführung des Verfahrens

Das erfindungsgemäße Verfahren benötigt zudem eine Base zur Deprotonierung des alkoxylierten Alkohols. Als Basen können dabei Alkalimetallhydoxide, Erdalkalimetallhydroxide sowohl als Feststoffe oder wässrige Lösungen eingesetzt werden. Wässrige Basen gelangen dabei üblicherweise in Konzentrationen von 10 Gew.-% bis hin zu gesättigten Lösungen mit suspendierten Feststoffen zum Einsatz. Besonders bevorzugt sind Lösungen zwischen 40 und 65 Gew.-%.

Das erfindungsgemäße Verfahren kann in einem weiten Temperaturbereich zwischen 10 und 140 °C ausgeführt werden. Im Hinblick auf eine ausreichende Reaktionsgeschwindigkeit einerseits und einem möglichst geringen Anteil an unerwünschten Nebenprodukten andererseits, empfiehlt sich ein Temperaturbereich von 40 bis 70 °C. Die Druckbedingungen sind bei dieser Reaktion nicht kritisch. Es kann sowohl bei Unterdruck, Normaldruck oder auch Überdruck gearbeitet werden.

Schließlich kann die Reaktion auch in Gegenwart eines inerten, organischen Lösemittels durchgeführt werden. Beispielhaft hierfür seien aromatische und aliphatische Kohlenwasserstoffe, Ether und andere dem Fachmann bekannte Lösemittel genannt. In der Regel kann der umzusetzende alkoxylierte Alkohol selbst als Lösungsmittel dienen.

## Patentansprüche

1. Verfahren zur Herstellung von unsymmetrischen alkoxylierten Mischethern der Formel (I)
R¹O(CH₂CH₂O)ₙ₁(CH₂CHR²O)ₘ(CH₂CH₂O)ₙ₂R³ (I)
in der R¹ für einen verzweigten Alkylrest mit 12 bis 16 Kohlenstoffatomen, R² für Methyl, R³ für einen Butylrest, n1 und n2 unabhängig voneinander für 0 oder Zahlen von 5 bis 15 sowie m für 0 oder Zahlen von 1 bis 20 mit der Maßgabe steht, dass die Summe (n1+n2+m) von 0 verschieden ist, durch Veretherung von Alkylpolyglykolethern der Formel (II)
R¹O(CH₂CH₂O)ₙ₁(CH₂CHR²O)ₘ(CH₂CH₂O)ₙ₂H (II)
in der R¹, R², n1, n2 und m die oben angegebene Bedeutung haben, mit 1-Chlorbutan **dadurch gekennzeichnet, dass** man die Umsetzung in Gegenwart von Phasen Transfer Katalysatoren (PTK) und Basen durchführt.

2. Verfahren nach Anspruch 1, **dadurch gekennzeichnet, dass** man Alkylpolyglykolether der Formel (I) einsetzt, in der R¹ für verzweigte Alkylreste mit 12 bis 16 Kohlenstoffatomen, R³ für einen Butylrest, n1 für Zahlen von 1 bis 15 und m und n2 für 0 stehen.

3. Verfahren nach den Ansprüchen 1 und/oder 2, **dadurch gekennzeichnet, dass** man die Alkylpolyglykolether und das 1-Chlorbutan im molaren Verhältnis 1:1 bis 1:1,5 einsetzt.

4. Verfahren nach mindestens einem der Ansprüche 1 bis 3, **dadurch gekennzeichnet dass** man PTK einsetzt, die ausgewählt sind aus der Gruppe, die gebildet wird von Tetraalkylammoniumsalzen, Benzyltrialkylammoniumsalzen, Tetraalkylphosphoniumsalzen, Benzyltrialkylphosphoniumsalzen und ihren Gemischen.

5. Verfahren nach mindestens einem der Ansprüche 1 bis 4, **dadurch gekennzeichnet, dass** man als PTK so genannte ionische Flüssigkeiten einsetzt.

6. Verfahren nach mindestens einem der Ansprüche 1 bis 5, **dadurch gekennzeichnet, dass** man die PTK in Mengen von 0,1 bis 25 Mol-% bezogen auf die Alkoholpolyglykolether einsetzt.

7. Verfahren nach mindestens einem der Ansprüche 1 bis 6, **dadurch gekennzeichnet, dass** man die PTK zusammen mit Co-Katalysatoren einsetzt, die ausgewählt sind aus der Gruppe der Alkali- und Erdalkalimetallcarboxylate, -carbonate und -thiocarboxylate.

8. Verfahren nach mindestens einem der Ansprüche 1 bis 7, **dadurch gekennzeichnet, dass** man die Veretherung bei Temperaturen im Bereich von 40 bis 140 °C durchführt.

## Claims

1. Process for the preparation of unsymmetric alkoxylated mixed ethers of the formula (I)
R¹O(CH₂CH₂O)ₙ₁(CH₂CHR²O)ₘ(CH₂CH₂O)ₙ₂R³ (I)
in which R¹ is a branched alkyl radical having 12 to 16 carbon atoms, R² is methyl, R³ is a butyl radical, n1 and n2, independently of one another, are 0 or numbers from 5 to 15, and m is 0 or numbers from 1 to 20, with the proviso that the sum (nl+n2+m) is different from 0, by etherification of alkyl polyglycol ethers of the formula (II)
R¹O (CH₂CH₂O)ₙ₁(CH₂CHR²O)ₘ(CH₂CH₂O)ₙ₂H (II)
in which R¹, R², n1, n2 and m have the meaning given above, with 1-chlorobutane, **characterized in that** the reaction is carried out in the presence of phase transfer catalysts (PTCs) and bases.

2. Process according to Claim 1, **characterized in that** alkyl polyglycol ethers of the formula (I) are used, in which R¹ is branched alkyl radicals having 12 to 16 carbon atoms, R³ is a butyl radical, n1 is numbers from 1 to 15 and m and n2 are 0.

3. Process according to Claims 1 and/or 2, **characterized in that** the alkyl polyglycol ethers and the 1-chlorobutane are used in a molar ratio of 1:1 to 1:1.5.

4. Process according to at least one of Claims 1 to 3, **characterized in that** PTCs are used which are selected from the group which is formed by tetraalkylammonium salts, benzyltrialkylammonium salts, tetraalkylphosphonium salts, benzyltrialkylphosphonium salts and their mixtures.

5. Process according to at least one of Claims 1 to 4, **characterized in that** so-called ionic liquids are used as PTCs.

6. Process according to at least one of Claims 1 to 5, **characterized in that** the PTCs are used in amounts of from 0.1 to 25 mol%, based on the alcohol polyglycol ethers.

7. Process according to at least one of Claims 1 to 6, **characterized in that** the PTCs are used together with co-catalysts which are selected from the group of the alkali metal and alkaline earth metal carboxylates, carbonates and thiocarboxylates.

8. Process according to at least one of Claims 1 to 7, **characterized in that** the etherification is carried out at temperatures in the range from 40 to 140°C.

## Revendications

1. Procédé pour la préparation d'éthers mixtes alcoxylés asymétriques de formule (I)
R¹O(CH₂CH₂O)ₙ₁(CH₂CHR²O)ₘ(CH₂CH₂O)ₙ₂R³ (I)
dans laquelle R¹ représente un radical alkyle ramifié comprenant 12 à 16 atomes de carbone, R² représente méthyle, R³ représente un radical butyle, n1 et n2 valent indépendamment l'un de l'autre 0 ou des nombres de 5 à 15 et m vaut 0 ou des nombres de 1 à 20, à condition que la somme (n1 + n2 + m) soit différente de 0, par éthérification d'alkylpolyglycoléthers de formule (II)
R¹O(CH₂CH₂O)ₙ₁(CH₂CHR²O)ₘ(CH₂CH₂O)ₙ₂H (II)
dans laquelle R¹, R², n1, n2 et m ont la signification indiquée ci-dessus, avec du 1-chlorobutane, **caractérisé en ce qu'**on réalise la transformation en présence de catalyseurs de transfert de phase (CTP) et de bases.

2. Procédé selon la revendication 1, **caractérisé en ce qu'**on utilise des alkylpolyglycoléthers de formule (I), dans laquelle R¹ représente des radicaux alkyle ramifiés comprenant 12 à 16 atomes de carbone, R³ représente un radical butyle, n1 vaut des nombres de 1 à 15 et m et n2 valent 0.

3. Procédé selon les revendications 1 et/ou 2, **caractérisé en ce qu'**on utilise les alkylpolyglycoléthers et le 1-chlorobutane dans un rapport molaire de 1:1 à 1:1,5.

4. Procédé selon au moins l'une quelconque des revendications 1 à 3, **caractérisé en ce qu'**on utilise des CTP qui sont choisis dans le groupe formé par les sels de tétraalkylammonium, de benzyltrialkylammonium, de tétraalkylphosphonium, de benzyltrialkylphosphonium et leurs mélanges.

5. Procédé selon au moins l'une quelconque des revendications 1 à 4, **caractérisé en ce qu'**on utilise comme CTP ce qu'on appelle des liquides ioniques.

6. Procédé selon au moins l'une quelconque des revendications 1 à 5, **caractérisé en ce qu'**on utilise les CTP en des quantités de 0,1 à 25% en mole par rapport aux alcoolpolyglycoléthers.

7. Procédé selon au moins l'une quelconque des revendications 1 à 6, **caractérisé en ce qu'**on utilise les CTP ensemble avec des cocatalyseurs, qui sont choisis dans le groupe des carboxylates, carbonates et thiocarboxylates de métal alcalin et de métal alcalino-terreux.

8. Procédé selon au moins l'une quelconque des revendications 1 à 7, **caractérisé en ce qu'**on réalise l'éthérification à des températures dans la plage de 40°C à 140°C.
